# EUROPEAN PATENT SPECIFICATION

(11) **EP 2 430 045 B1**
(45) Date of publication and mention of the grant of the patent: **08.07.2015**
(21) Application number: 10774609.1
(22) Date of filing: 12.05.2010
(51) Int. Cl.: C07K 16/08, C12Q 1/70, C07K 16/10

(54) **CARBOHYDRATE BINDING MODULE AND USE THEREOF**
KOHLENHYDRATBINDENDES MODUL UND SEINE VERWENDUNG
MODULE DE LIAISON AU GLUCIDE ET SON UTILISATION

(30) Priority: 12.05.2009 US 464788
(43) Date of publication of application: 21.03.2012
(73) Proprietor: National Tsing Hua University, Hsinchu 30013 (TW)
(72) Inventor: CHANG, Margaret Dah-tsyr, Hsinchu 30013 (TW); LEE, Yuan-chuan, Hsinchu 30013 (TW); HUANG, Rong-yuan, Hsinchu 30013 (TW); LIN, Shu-chuan, Hsinchu 30013 (TW); CHOU, Wei-i, Hsinchu 30013 (TW); LIU, Shi-hwei, Hsinchu 30013 (TW)
(74) Representative: Ter Meer Steinmeister & Partner
(86) International application number: PCT/IB2010/001302
(87) International publication number: WO 2010/131114

(56) References cited:
- US-A1- 2005 090 643
- US-A1- 2007 116 651
- US-A1- 2009 297 516
- BARRIENTOS LAURA G ET AL: "THE HIGHLY SPECIFIC CARBOHYDRATE-BINDING PROTEIN CYANOVIRIN-N: STRUCTURE, ANTI-HIV/EBOLA ACTIVITY AND POSSIBILITIES FOR THERAPY", MINI REVIEWS IN MEDICINAL CHEMISTRY, BENTHAM SCIENCE PUBLISHERS, HILVERSUM, NL, vol. 5, no. 1, 1 January 2005 (2005-01-01) , pages 21-31, XP009073867, ISSN: 1389-5575
- Tsunoda et al.: "Crystal structure of actinohivin; A novel anti-human immunodeficiency virus protein", , 1 January 2006 (2006-01-01), XP055048237, Retrieved from the Internet: URL:http://journals.iucr.org/a/issues/2008 /a1/00/a38962/a38962.pdf [retrieved on 2012-12-18]
- BYOUNG CHAN KIM ET AL: "Quantitative Detection of HIV-1 Particles Using HIV-1 Neutralizing Antibody-Conjugated Beads", ANALYTICAL CHEMISTRY, vol. 81, no. 6, 15 March 2009 (2009-03-15) , pages 2388-2393, XP055048504, ISSN: 0003-2700, DOI: 10.1021/ac802267u
- BOTOS ET AL.: 'Proteins that bind high-mannose sugars of the HIV envelope.' PROG BIOPHYS MOL BIOL vol. 88, no. 2, June 2005, pages 233 - 282, XP027691178
- MACHOVIC ET AL.: 'A new clan of CBM families based on bioinformatics of starch-binding domains from families CBM20 and CBM21.' FEBS J vol. 272, no. 21, November 2005, pages 5497 - 5513, XP002374813
- CHRISTIANSEN ET AL.: 'The carbohydrate-binding module family 20-diversity, structure, and function.' FEBS J vol. 276, no. 18, September 2009, pages 5006 - 5029, XP002669193
- CALARESE ET AL.: 'Dissection of the carbohydrate specificity of the broadly neutralizing anti-HIV- 1 antibody 2G12.' PROC NAT ACAD SCI vol. 102, no. 38, 20 September 2005, pages 13372 - 13377, XP002595010

### SEQUENCE LISTING

<110> National Tsing Hua University
<120> ANTI-VIRAL CARBOHYDRATE BINDING MODULE COMPOSITIONS AND METHODS OF USE
<130> 1178-NTHU-US
<160> 22
<170> PatentIn version 3.5
<210> 1
   <211> 24
   <212> DNA
   <213> Artificial Sequence
<220>
   <223> forward primer for pET23a(+)-RoSBD
<220>
   <221> primer_bind
   <222> (1)..(24)
<400> 1
   catatggcaa gtattcctag cagt 24
<210> 2
   <211> 23
   <212> DNA
   <213> Artificial Sequence
<220>
   <223> reverse primer for pET23a(+)-RoSBD
<220>
   <221> primer_bind
   <222> (1)..(23)
<400> 2
   ctcgagtcat gtagatactt ggt 23
<210> 3
   <211> 24
   <212> DNA
   <213> Artificial Sequence
<220>
   <223> forward primer for pET15b-AnSBD
<220>
   <221> primer_bind
   <222> (1)..(24)
<400> 3
   catatgagca agaccagcac cagt 24
<210> 4
   <211> 20
   <212> DNA
   <213> Artificial Sequence
<220>
   <223> reverse primer for pET15b-AnSBD
<220>
   <221> primer_bind
   <222> (1)..(10)
<400> 4
   ctcgagctac cgccaggtgt 20
<210> 5
   <211> 30
   <212> DNA
   <213> Artificial Sequence
<220>
   <223> An oligonucleotide primer for site-directed mutagenesis-N29A-F'
<220>
   <221> primer_bind
   <222> (1)..(30)
<400> 5
   atttatgtca aggccattgc ttactccaag 30
<210> 6
   <211> 33
   <212> DNA
   <213> Artificial Sequence
<220>
   <223> An oligonucleotide primer for site-directed mutagenesis-N29A-R'
<220>
   <221> primer_bind
   <222> (1)..(33)
<400> 6
   ggagtaagca atggccttga cataaatttt tcc 33
<210> 7
   <211> 33
   <212> DNA
   <213> Artificial Sequence
<220>
   <223> An oligonucleotide primer for site-directed mutagenesis-Y32A-F'
<220>
   <221> primer_bind
   <222> (1)..(33)
<400> 7
   gtcaagaaca ttgctgcctc caagaaagtt act 33
<210> 8
   <211> 33
   <212> DNA
   <213> Artificial Sequence
<220>
   <223> An oligonucleotide primer for site-directed mutagenesis-Y32A-R'
<220>
   <221> primer_bind
   <222> (i)..(33)
<400> 8
   agtaactttc ttggaggcag caatgttctt gac 33
<210> 9
   <211> 31
   <212> DNA
   <213> Artificial Sequence
<220>
   <223> An oligonucleotide primer for site-directed mutagenesis-K34A-F'
<220>
   <221> primer_bind
   <222> (1)..(31)
<400> 9
   cattgcttac tccgcgaaag ttactgtagt c 31
<210> 10
   <211> 30
   <212> DNA
   <213> Artificial Sequence
<220>
   <223> An oligonucleotide primer for site-directed mutagenesis-K34A-R'
<220>
   <221> primer_bind
   <222> (1)..(30)
<400> 10
   gactacagta actttcgcgg agtaagcaat 30
<210> 11
   <211> 33
   <212> DNA
   <213> Artificial Sequence
<220>
   <223> An oligonucleotide primer for site-directed mutagenesis-W47A-F'
<220>
   <221> prim_transcript
   <222> (1)..(33)
<400> 11
   gatggctctg acaacgcgaa taataatgga aac 33
<210> 12
   <211> 33
   <212> DNA
   <213> Artificial Sequence
<220>
   <223> An oligonucleotide primer for site-directed mutagenesis-W47A-R'
<220>
   <221> primer_bind
   <222> (1)..(33)
<400> 12
   gtttccatta ttattcgcgt tgtcagagcc atc 33
<210> 13
   <211> 23
   <212> DNA
   <213> Artificial Sequence
<220>
   <223> An oligonucleotide primer for site-directed mutagenesis-F58A-F'
<220>
   <221> primer_bind
   <222> (1)..(23)
<400> 13
   catcattgct gcttctgcct ctg 23
<210> 14
   <211> 22
   <212> DNA
   <213> Artificial Sequence
<220>
   <223> An oligonucleotide primer for site-directed mutagenesis-F58A-R'
<220>
   <221> primer_bind
   <222> (1)..(22)
<400> 14
   agagataggg ccagaggcag aa 22
<210> 15
   <211> 25
   <212> DNA
   <213> Artificial Sequence
<220>
   <223> An oligonucleotide primer for site-directed mutagenesis-Y67A-F'
<220>
   <221> primer_bind
   <222> (1)..(25)
<400> 15
   ctctggatca aatgccgaat actgg 25
<210> 16
   <211> 25
   <212> DNA
   <213> Artificial Sequence
<220>
   <223> An oligonucleotide primer for site-directed mutagenesis-Y67A-R'
<220>
   <221> primer_bind
   <222> (1)..(25)
<400> 16
   atgtccagta ttcggcattt gatcc 25
<210> 17
   <211> 21
   <212> DNA
   <213> Artificial Sequence
<220>
   <223> An oligonucleotide primer for site-directed mutagenesis-Y83A-F'
<220>
   <221> primer_bind
   <222> (1)..(21)
<400> 17
   ggtaccatta agtatgaagt c 21
<210> 18
   <211> 33
   <212> DNA
   <213> Artificial Sequence
<220>
   <223> An oligonucleotide primer for site-directed mutagenesis-Y83A-R'
<220>
   <221> primer_bind
   <222> (1)..(33)
<400> 18
   gacttcatac ttaatggcga actccttgat acc 33
<210> 19
   <211> 33
   <212> DNA
   <213> Artificial Sequence
<220>
   <223> An oligonucleotide primer for site-directed mutagenesis-Y93A-F'
<220>
   <221> primer_bind
   <222> (1)..(33)
<400> 19
   gtcagtggaa aaacagccta tgataacaac aat 33
<210> 20
   <211> 33
   <212> DNA
   <213> Artificial Sequence
<220>
   <223> An oligonucleotide primer for site-directed mutagenesis-Y93A-R'
<220>
   <221> primer_bind
   <222> (1)..(33)
<400> 20
   attgttgtta tcataggctg tttttccact gac 33
<210> 21
   <211> 31
   <212> DNA
   <213> Artificial Sequence
<220>
   <223> An oligonucleotide primer for site-directed mutagenesis-Y94A-F'
<220>
   <221> primer_bind
   <222> (1)..(31)
<400> 21
   gtggaaaaac atacgctgat aacaacaatt c 31
<210> 22
   <211> 31
   <212> DNA
   <213> Artificial Sequence
<220>
   <223> An oligonucleotide primer for site-directed mutagenesis-**Y94A**-R'
<220>
   <221> primer_bind
   <222> (11)..(31)
<400> 22
   gaattgttgt tatcagcgta tgtttttcca c 31

## Description

### FIELD OF THE INVENTION

The present invention relates to an antibody mimetic of carbohydrate binding module (CBM) which specifically binds to an epitope on HIV glycoprotein. The present invention also relates to a method of inhibiting HIV activity.

### BACKGROUND OF THE INVENTION

Human immunodeficiency virus (HIV) is known to cause acquired immune deficiency syndrome (AIDS), and because the HIV exhibits rapid genetic drift, widely divergent strains are emerging. Thus, detection and treatment of variant strains have proven to be challenging and difficult.

There is also an urgent need to develop an effective prophylactic vaccine and other therapeutic strategies to limit HIV transmission as the epidemic continuous unabated. Most successful vaccines consist of either live-attenuated or inactivated viral particles. However, live-attenuation of the HIV-related simian immunodeficiency virus, resulting in protective responses without resulting pathogenicity has not been accomplished, raising safety concerns that make human trial intractable. Also, HIV has many sophisticated mechanisms to evade envelope glycoprotein-directed antibody responses efficiently, including shrouding well-conserved structures by glycan shielding and masking of vulnerable receptor-binding sites by conformational and steric constraints. Therefore, researches turned to envelope glycoprotein-based immunogens as a means of elicitng antibodies. Glycoprotein 120 (gp120) is an important component of the envelope spikes of HIV-1. The inner domain of gp120 interacts with gp41, and the outer domain is quite variable and heavily glycosylated (Kwong, P.D. et al. Structure of an HIV gp120 envelope glycoprotein in complex with the CD4 receptor and a neutralizing human antibody. Nature 393, 648-659 (1998)). Based on comparative sequence analysis, gp120 is divided into five conserved segments (C1 to C5) and five variable (V1 to V5) regions (Willey, R.L. et al. Identification of conserved and divergent domains within the envelope gene of the acquired immunodeficiency syndrome retrovirus. Proc Natl Acad Sci U S A 83, 5038-5042 (1986) and Modrow, S. et al. Computer-assisted analysis of envelope protein sequences of seven human immunodeficiency virus isolates: prediction of antigenic epitopes in conserved and variable regions. J Virol 61, 570-578 (1987)). The C1 and C5 regions provide the main areas for contact of gp120 with gp41 (Helseth, E., Olshevsky, U., Furman, C. & Sodroski, J. Human immunodeficiency virus type 1 gp120 envelope glycoprotein regions important for association with the gp41 transmembrane glycoprotein. J Virol 65, 2119-2123 (1991)), the other conserved regions (C2 to C4) form a hydrophobic core within the gp120 molecule (Moore, J.P., Sattentau, Q.J., Wyatt, R. & Sodroski, J. Probing the structure of the human immunodeficiency virus surface glycoprotein gp120 with a panel of monoclonal antibodies. J Virol 68, 469-484 (1994) and Moore, J.P., Willey, R.L., Lewis, GK., Robinson, J. & Sodroski, J. Immunological evidence for interactions between the first, second, and fifth conserved domains of the gp120 surface glycoprotein of human immunodeficiency virus type 1. J Virol 68, 6836-6847 (1994)) and the variable regions, V1 to V3, are exposed on the surface of monomeric gp120, and possess major epitopes for antibody recognition and binding sites for CD4 (Chen, L. et al. Structural basis of immune evasion at the site of CD4 attachment on HIV-1 gp120. Science 326, 1123-1127 (2009), Moore, J.P. & Sodroski, J. Antibody cross-competition analysis of the human immunodeficiency virus type 1 gp120 exterior envelope glycoprotein. J Virol 70, 1863-1872 (1996), Pollard, S.R., Rosa, M.D., Rosa, J.J. & Wiley, D.C. Truncated variants of gp120 bind CD4 with high affinity and suggest a minimum CD4 binding region. EMBO J 11, 585-591 (1992) and Olshevsky, U. et al. Identification of individual human immunodeficiency virus type 1 gp120 amino acids important for CD4 receptor binding. J Virol 64, 5701-5707 (1990)).

At present, 47 cases of neutralizing monoclonal antibodies (mAbs) recognizing HIV have entered into phase I or II clinical trials in the United States. Among these, human mAbs 2F5, 4E10, and 2012 have been well characterized in their recognition of HIV1 envelope glycoproteins (Armbruster, C. et al. A phase I trial with two human monoclonal antibodies (hMAb 2F5, 2G12) against HIV-1. AIDS 16, 227-233 (2002) and Stiegler, G et al. Antiviral activity of the neutralizing antibodies 2F5 and 2G12 in asymptomatic HIV-1-infected humans: a phase I evaluation. AIDS 16, 2019-2025 (2002)). Most of these antibodies primarily target regions of gp120 which are occluded in its mature trimeric form on the viral surface (Zwick, M.B. & Burton, D.R. HIV-1 neutralization: mechanisms and relevance to vaccine design. Curr HIV Res 5, 608-624 (2007) and Krauss, I.J. et al. Fully synthetic carbohydrate HIV antigens designed on the logic of the 2G12 antibody. J Am Chem Soc 129, 11042-11044 (2007)). The remaining exposed faces of gp120 are heavily glycosylated with *N*-linked high mannose glycan Man9-GlcNAc (M9), and few antibodies have been isolated to successfully bind to such "immunologically silent" regions. Human mAb 2G12 was however reported to possess neutralizing activity against clades A and B of HIV-1 (Trkola, A. et al. Human monoclonal antibody 2G12 defines a distinctive neutralization epitope on the gp120 glycoprotein of human immunodeficiency virus type 1. J Virol 70, 1100-1108 (1996)) and binds to an epitope on the "silent face" of HIV-1 gp120. Alanine scanning studies employing site-directed mutagenesis showed that the epitope predominantly covered the high-mannose or hybrid glycan structures of residues N295, N332, N386, N392, N397 and N448 on gp120 (Sanders, R.W. et al. The mannose-dependent epitope for neutralizing antibody 2G12 on human immunodeficiency virus type 1 glycoprotein gp120. J Virol 76, 7293-7305 (2002) and Scanlan, C.N. et al. The broadly neutralizing anti-human immunodeficiency virus type 1 antibody 2G12 recognizes a cluster of alphal-->2 mannose residues on the outer face of gp120. J Virol 76, 7306-7321 (2002)), unlike most antibodies recognize a specific configuration of amino acid side chains. The possibility therefore exists that 2G12 could be turned into a broad-ranging HIV neutralizing antibody based on recognition of carbohydrate epitopes.

The crystal structure of 2G12 (PDB ID: 1ZLS) and its complex with the oligosaccharide M9 (PDB ID: 1OP5) revealed that two Fabs assembled into a V_{H} domain-swapped dimer, and the V_{H} domain located on the heavy chain of 2G12 interacted directly with the *N*-linked M9, especially the D1 arm comprising a tetramannose Man_{α(1},₂₎Man_{α(1},₂₎Man_{α(1,3)}Man_{α} (Man4) moiety, on gp120. In addition, molecular modeling of the 2G12 recognition sites on gp120 has shown that the *N*-linked glycans on N332, N339, and N392 were critical for 2G12 binding (Calarese, D.A. et al. Antibody domain exchange is an immunological solution to carbohydrate cluster recognition. Science 300, 2065-2071 (2003)). The synthetic oligosaccharides Man4 and Man9 could inhibit the binding between gp120 and 2G12, hence these oligomannose dendrions were identified as potential HIV1 neutralizing agents. Moreover, the IC₅₀ neutralization profiles of several HIV mAbs reveal that the neutralizing ability of 2G12 is moderate (Binley, J.M. et al. Comprehensive cross-clade neutralization analysis of a panel of anti-human immunodeficiency virus type 1 monoclonal antibodies. J Virol 78, 13232-13252 (2004)).

According to the Protein Structure Classification Database (CATH) (Orengo, C.A. et al. CATH--a hierarchic classification of protein domain structures. Structure 5, 1093-1108 (1997)), the V_{H} domain of 2G12 is categorized into superfamily 2.60.40.10 with characteristic features of the β-sandwich fold, immunoglobulin-like (Ig-like) structure. Interestingly, a granular starch binding domain (SBD) of *Aspergillus niger* glucoamylase, *An*SBD, (PDB ID: 1AC0) has also been identified in the same homologous superfamily (Christiansen, C. et al. The carbohydrate-binding module family 20--diversity, structure, and function. FEBS J 276, 5006-5029 (2009)). The structural similarity between 2G12 and such a carbohydrate-binding module (CBM) may imply common functions as an effective detection and prophylactic vaccine which could be elucidated.

### SUMMARY OF THE INVENTION

The present invention provides a method of inhibiting prophylactically or therapeutically HIV infection of a subject, wherein the method comprises administering to the subject an effective amount of an antibody mimetic of carbohydrate binding module (CBM) which specifically binds to an epitope on HIV glycoprotein.

The present invention further provides a pharmaceutical composition comprising a therapeutically or prophylactically effective amount of an antibody mimetic of carbohydrate binding module and a pharmaceutically acceptable carrier.

### BRIEF DESCRIPTION OF THE DRAWINGS

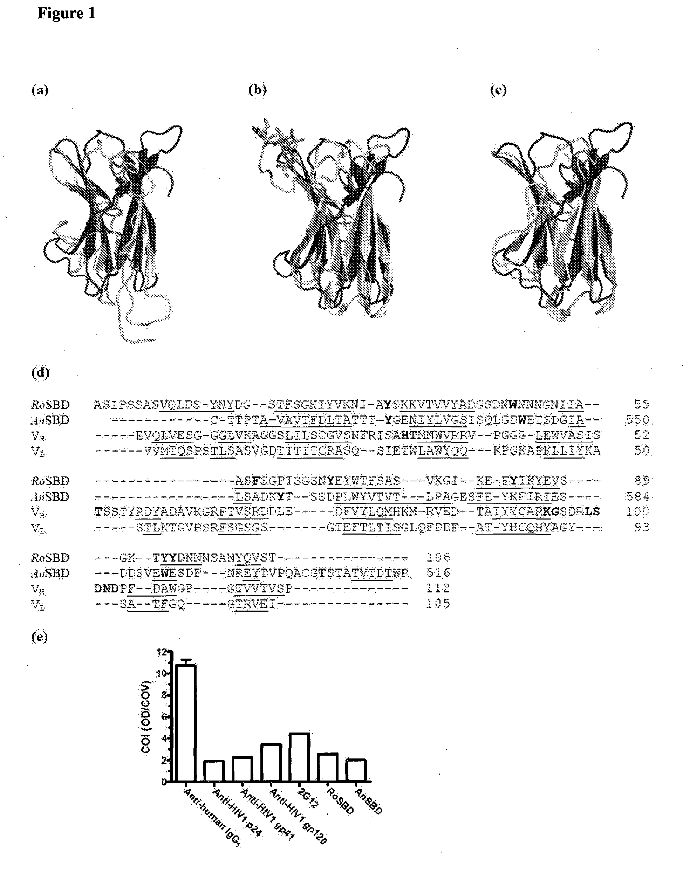

**Figure 1****.** Comparison of structure, sequence and function for starch-binding CBMs and 2G12. The structural superimposition of *RoS*BD (dark gray) with: **(a)** *An*SBD (light gray); **(b)** 2G12 V_{H} domain (light gray) and **(c)** 2G12 V_{L} domain (light gray). Man4 in **(b)** is shown in stick representation. **(d)** Multiple sequence alignment of *Ro*SBD, *An*SBD, 2G12 V_{H} and 2G12 V_{L} based on the outcome of Dali superimposition. Underlined residues denote β-strands regions, and amino acids in boldface denote the ligand binding residues in the SBDs and Man4-binding sites in 2G12 V_{H} domain. **(e)** One hundred microliters of HIV-PC solutions were loaded into 96-well ELISA plates pre-coated separately with 100 nM human IgG₁ secondary antibody (2C11), HIV-1 p24 mAb (N29), HIV-1 gp41 mAb (AG10H9), HIV-1 gp120 mAb (ED8.D4), 2G12, *Ro*SBD and *An*SBD. HRP-conjugated recombinant gp120 antigen was used for signal detection. The difference was statistically significant (P < 0.0001).

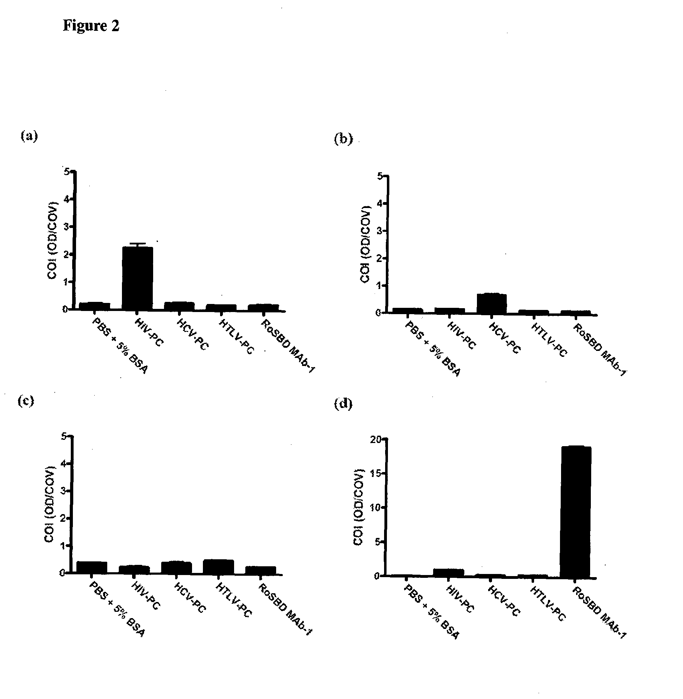

**Figure 2****.** Specificity of *Ro*SBD-HIV binding. One hundred microliters of HIV-PC, *Ro*SBD mAb and HCV-PC were mixed together, loaded onto *Ro*SBD-coated ELISA plates, and allowed to react at 37 °C for 1 h. Detection was achieved by addition of 100 µL of: **(a)** HRP-conjugate recombinant gp120 antigen; **(b)** HRP-conjugate HCV recombinant antigen; **(c)** HRP-conjugate HTLV recombinant antigen or **(d)** Anti-mouse IgG HRP conjugate. Here the cut off value (COV) was defined as the absorbence value obtained in the ELISA assay from normal human serum plus 0.1, and the maximal cut off index (COI) value was calculated by dividing OD value with COV. The difference was statistically significant (P < 0.05).

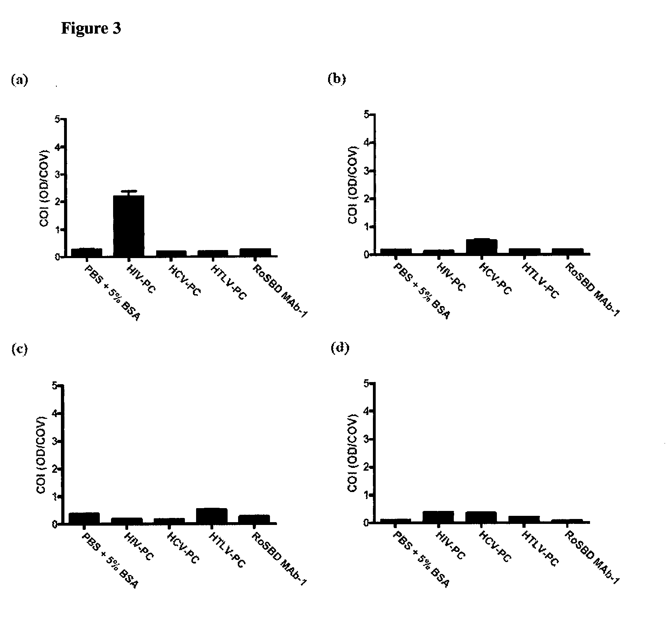

**Figure 3****.** Specificity of *An*SBD-HIV binding. One hundred microliters of HIV-PC, *Ro*SBD mAb and HCV-PC were mixed together, loaded onto *An*SBD-coated ELISA plates, and allowed to react at 37 °C for 1 h. Detection was achieved by addition of 100 µL of: **(a)** HRP-conjugate recombinant gp120 antigen; **(b)** HRP-conjugate HCV recombinant antigen; **(c)** HRP-conjugate HTLV antigen or **(d)** HRP-conjugate anti-mouse IgG . Here the cut off value (COV) was defined as the absorbence value obtained in the ELISA assay from normal human serum plus 0.1, and the maximal cut off index (COI) value was calculated by dividing OD value with COV. The difference was statistically significant (P < 0.0001).

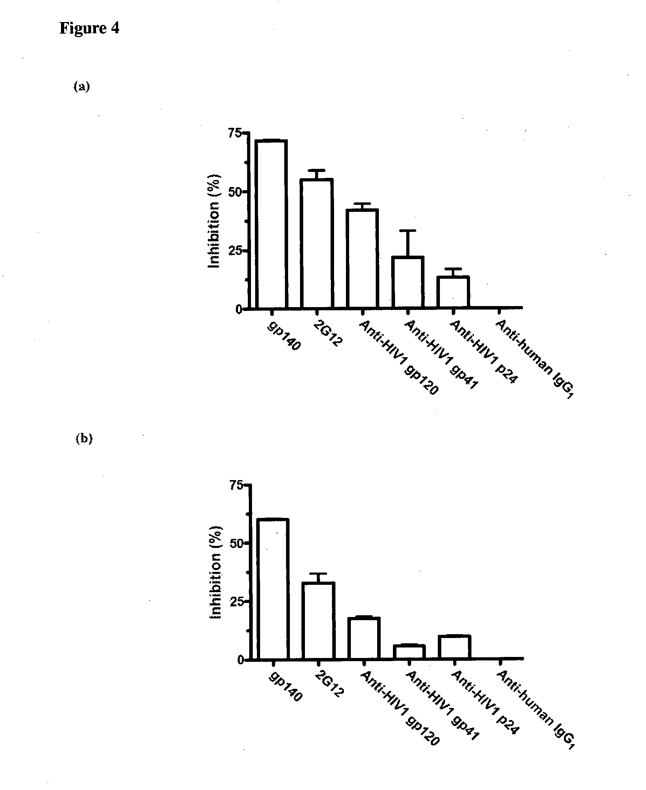

**Figure 4****.** Competitive binding of HIV to SBDs. HIV-PC was separately mixed with the same volume of 500 nM gp140, 2G12 mAb, gp120 mAb (ED8.D4), gp41 mAb (AG10H9), p24 mAb (N29), or human IgG₁ secondary antibody (2C11) at 37 °C for 1 h. The mixture was transferred to a plate coated with **(a)** *Ro*SBD or **(b)** *An*SBD and detected with HRP-conjugated recombinant gp120 antigen. The difference was statistically significant (P < 0.0001).

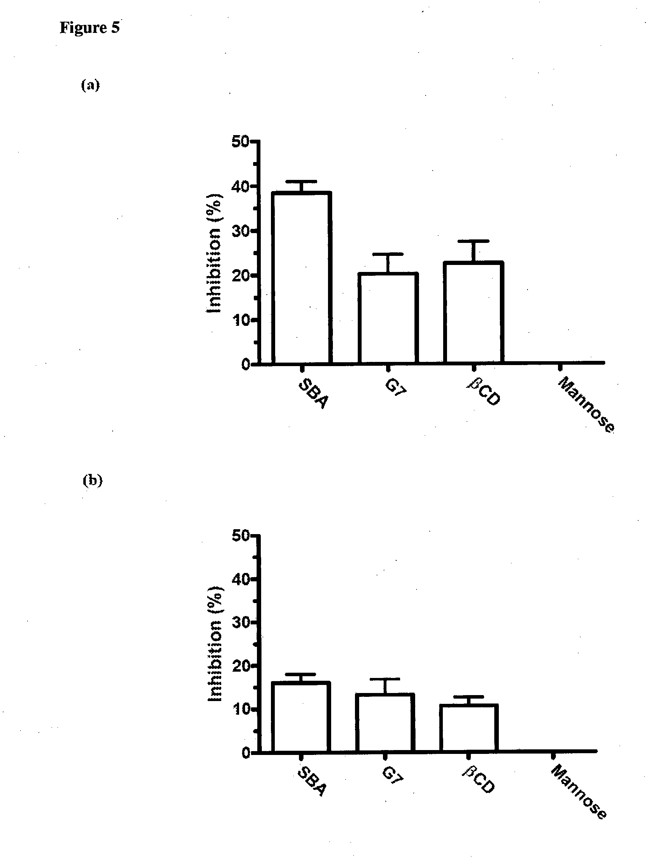

**Figure 5****.** Competitive binding of glycans. HIV-PC was mixed with one each of 500 µM soybean agglutinin (SBA), G7, βCR, or mannose at 37 °C for 1 h. The mixture was transferred to an ELISA plate pre-coated with 100 nM of: **(a)** *Ro*SBD and **(b)** *An*SBD. The reaction was detected using HRP-conjate recombinant gp120 antigen. The difference was statistically significant (P < 0.01).

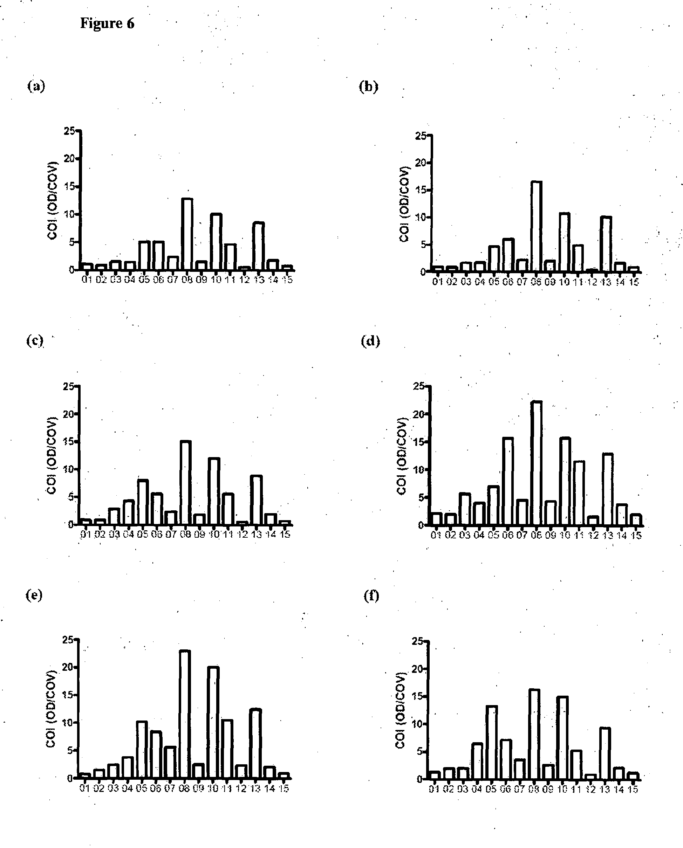

**Figure 6****.** HIV binding profiles using mAbs and SBDs 100 µL of HIV-1 incidence/prevalence performance panel (PRB601) members were separately loaded onto an ELISA plate pre-coated with 100 nM of: **(a)** HIV-1-p24 mAb (N29); **(b)** HIV-1-gp41 mAb (AG10H9); **(c)** gp120 mAb (ED8.D4); **(d)** HIV-1-2G12 mAb; **(e)** *Ro*SBD or **(f)** *An*SBD and detected with HRP-conjugate recombinant gp120 antigen. Here the cut off value (COV) was defined as the absorbence value obtained in the ELISA assay from normal human serum plus 0.1, and the maximal cut off index (COI) value was calculated by dividing OD value with COV.

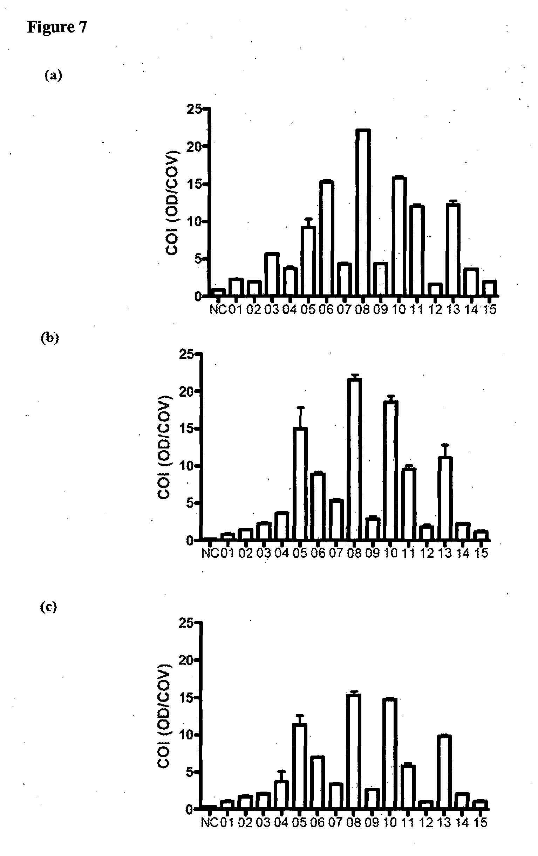

**Figure 7****.** HIV-2G12 and HIV-SBD binding profiles. One hundred microliters of HIV-1 performance panel (PRB601) members were separately loaded into 96-well ELISA plate that were *pre-coated* with 100 nM **(a)** 2G12, **(b)** *Ro*SBD and **(c)** *An*SBD. The reaction was detected using HRP-conjugate recombinant gp120 antigen. PBS containing 5% BSA (pH 7.4) was used as NC Negative Control (NC). The cut off value (COV) was defined as the absorbance value obtained in the ELISA assay from normal human serum plus 0.1, and the maximal cut off index (COI) value was calculated by dividing OD value with COV. The difference was statistically significant (P < 0.0001).

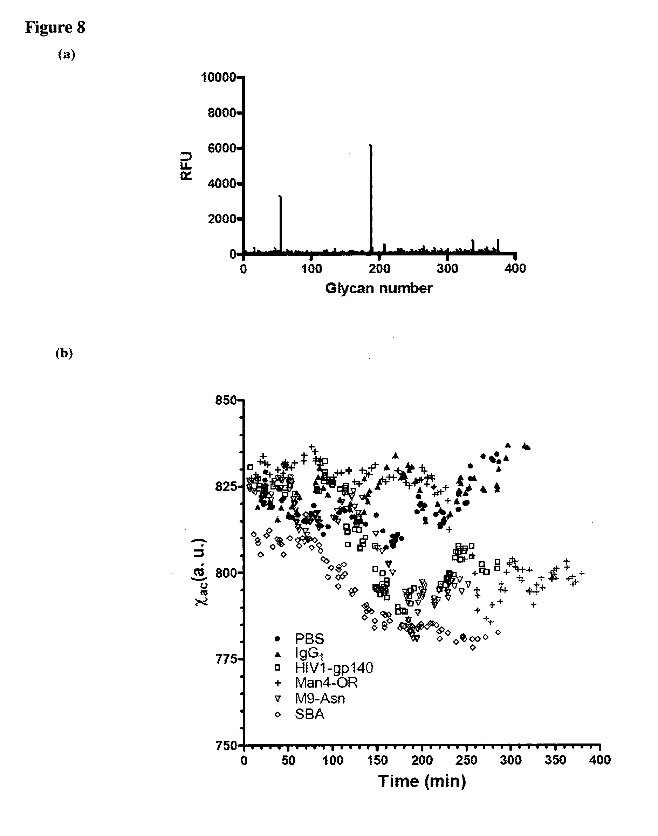

**Figure 8****.** Glycan binding activities of *Ro*SBD. **(a)** Glycan microarray analyses were conducted by the Consortium for Functional Glycomics, Core H facility. The primary antibody used in the analysis was anti-*Ro*SBD monoclonal antibody, and the secondary antibody was Alexa Fluor 488-conjugated goat anti-mouse IgG. **(b)** Sixty microliters of 0.7 pM IgG1 (2C11), HIV-1 gp140, Man4-OR, or Man9-Asn in PBS (pH 7.4) was placed in a glass tube, and 60 µL magnetic reagent was added to the glass tube and mixed for 15 sec at 4 °C prior to magnetic reduction measurement. The magnetic susceptibility of the mixture was measured at 25 °C using a XacPro-S101 reader.

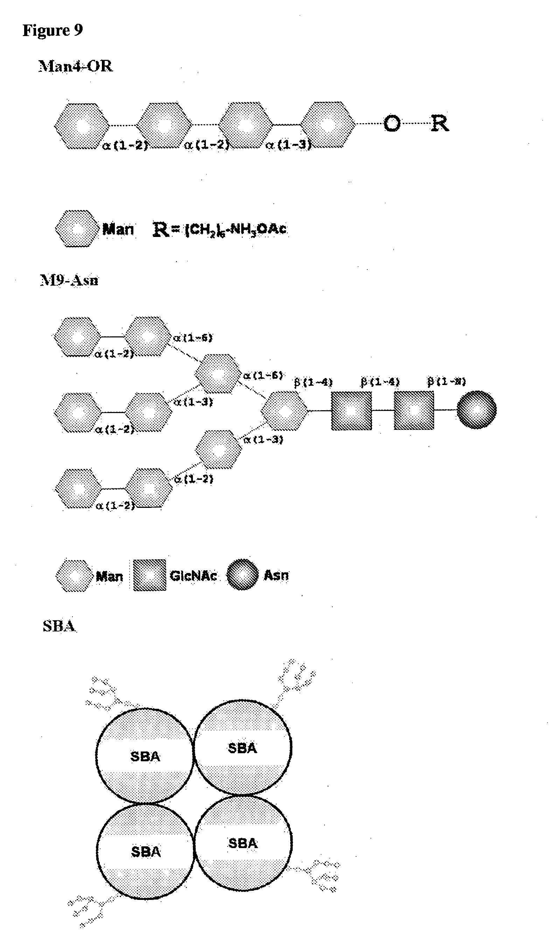

**Figure 9****.** Structures of synthetic Man4 (as 6-aminohexyl glycoside Man4-OR) and M9-Asn (from SBA).

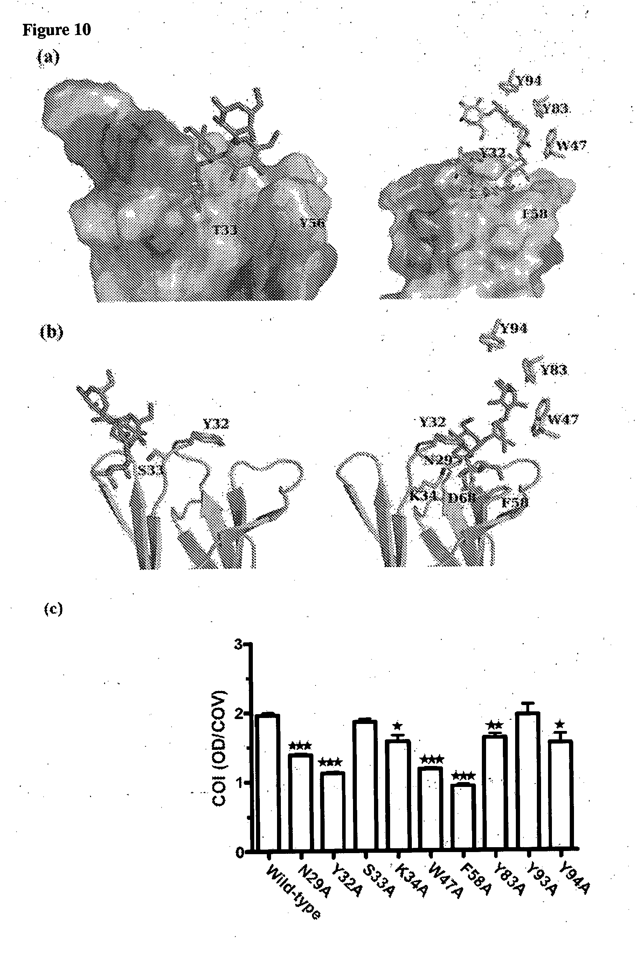

**Figure 10****.** Man4 binding sites on *Ro*SBD. **(a)** Surface potentials calculated and displayed using PyMOL, showing negative (red), and positive (blue) potentials. Left panel: The V_{H}-Man4 with residues Thr33, Tyr56 and Asp100 labeled, and Man4 shown in brown. Right panel: The *Ro*SBD-G7 complex showing labels for the key binding residues of site I (Trp47, Tyr83, and Tyr94) and site II (Tyr32 and Phe58). G7 molecules are shown in cyan. **(b)** Ribbon diagrams of predicted Man4 binding site on *Ro*SBD. Man4 molecules are shown in brown. Left panel: Residues Tyr32, Ser33 and Lys34 are shown. Right panel: Residues Asn29, Tyr32, Lys34, Trp47, Phe58 and Asp68 are shown. **(c)** HIV-binding of wild-type and mutant *Ro*SBD. 100 µL HIV-PC solutions were loaded into 96-well ELISA *plates pre-coated* with 100 nM wild-type *Ro*SBD or mutant *Ro*SBD N29A, Y32A, S33A, K34A, W47A, F58A, Y83A, Y93A, and Y94A. HRP-conjugate recombinant gp120 antigen was used for signal detection. Results represented means ± SD of triplicate samples. ★, p<0.05; **, p<0.01; ★★★, p<0.0001.

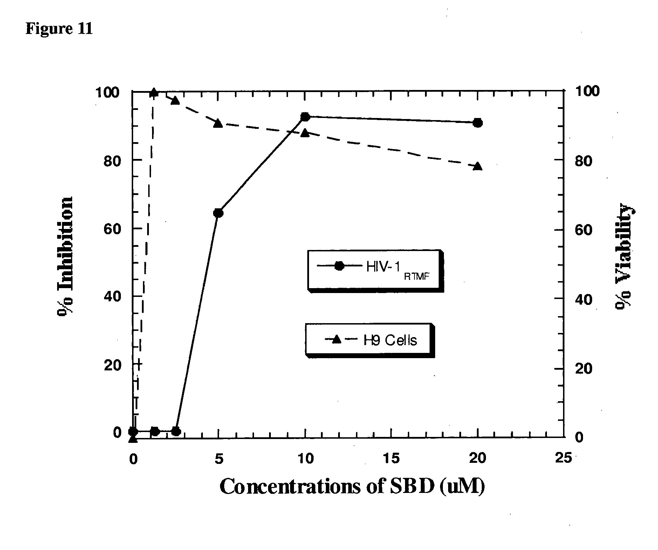

**Figure 11****.** Effect of *Ro*SBD amino acids on HIV-1 replication and cell viability. HIV-1_{RTMF} (AZT-resistant virus), was treated with increasing concentrations of *Ro*SBD. Viral replication was measured by using the HIV-1 p24 antigen ELISA after 8 days and the IC₅₀ value for the inhibition was calculated from the dose response curve. The effect of *Ro*SBD on H9 cell viability with various concentrations and MTT assay were used to determine the cytotoxicity in H9 cell culture.

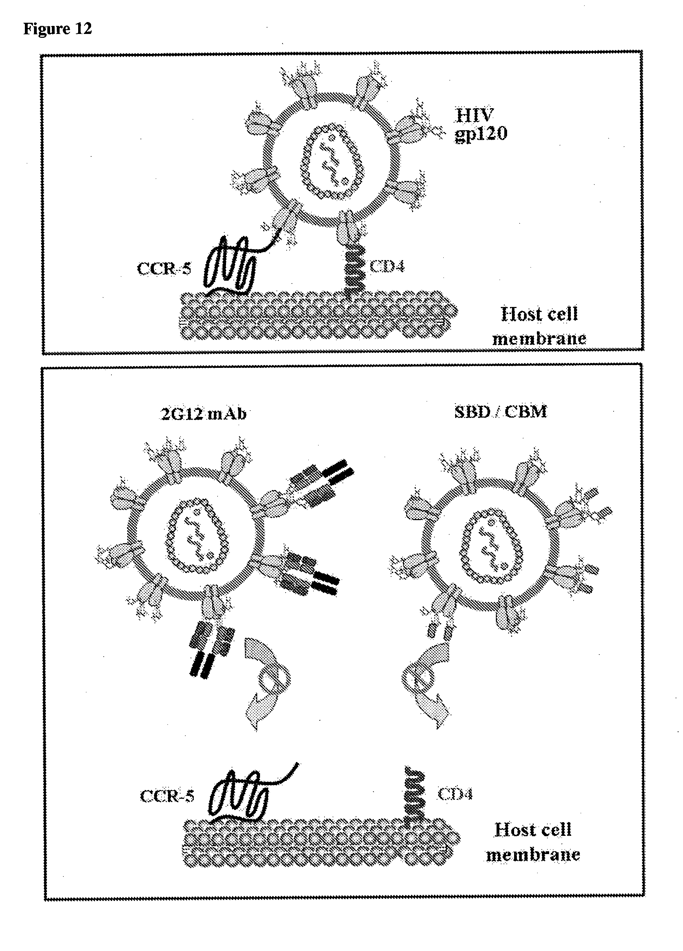

**Figure 12****.** Possible strategies for inhibiting HIV-1 entry. **(a)** The entry of HIV-1 into target cells requires binding of the gp120 with cell surface receptor CD4 and co-receptor CCR-5. **(b)** The neutralizing mAb 2G12 blocks HIV-1 infection by recognizing the CD4 binding domain of HIV gp120. **(c)** The SBD/CBM may recognize HIV-1 and act as a mimic of 2G12 mAb to block HIV-1 infection.

### DETAILED DESCRIPTION OF THE INVENTION

The present invention provides a method of inhibiting prophylactically or therapeutically HIV infection of a subject, comprising administering to the subject an effective amount of an antibody mimetic of carbohydrate binding module (CBM) which specifically binds to an epitope on HIV glycoprotein.

The term "CBM" used herein refers as a contiguous amino acid sequence within a carbohydrate-active enzyme with a discreet fold having carbohydrate-binding activity. In the primary structure classification of glycoside hydrolases, the CBMs are categorized into 59 families on the basis of amino acid sequence similarity and they exhibit diverse ligand specificity, according to the Carbohydrate-Active EnZyme (CAZy) database (Cantarel, B.L. et al. The Carbohydrate-Active EnZymes database (CAZy): an expert resource for Glycogenomics. Nucleic Acids Res 37, D233-238 (2009)), which include several specificities such as cellulose, xylan, chitin, and starch binding. In the present invention, the CBMs specifically bind to a glycan structure present in the epitope of the HIV glycoprotein. In a preferable embodiment, the HIV glycoprotein is gp120, and the the glycan structure is N-linked high mannose glycan Man9-GlcNAc (M9).

The term "antibody mimetic" used herein refers to an object having similar function as the antibody in terms of binding against target structure, but its structure is simpler than an antibody. To produce a large amount of antibodies needs the following steps: (a) fusing single antibody-forming cells to tumor cells grown in culture. The resulting cell is called a hybridoma, (b) each hybridoma produces relatively large quantities of identical antibody molecules, and (c) allowing the hybridoma to multiply in culture, it is possible to produce a population of cells, each of which produces identical antibody molecules. It is labor-consuming and cost-consuming to make real antibodies; however, in the present invention, a broad range of host including bacteria, yeast, insect, and mammalian cells can be used to produce the antibody mimetic of CBM without using animals, which is simpler and more economic.

The term "subject" as used herein relates to animals, preferably mammals, and, more preferably, humans.

In the present invention, the CBM is starch binding domain (SBD). The term "SBD" used herein refers to a functional domain that can bind granular or soluble starch, increasing the local concentration of substrate at the active site of the enzyme, and that may also disrupt the structure of the starch surface, thereby enhancing the amylolytic rate. At present, there are nine starch-binding CBM families: CBM20, CBM21, CBM25, CBM26, CBM34, CBM41, CBM45, CBM48 and CBM53. In a preferred embodiment of the invention, the SBD is the member of the CBM families 20 and 21, which is derived from *Aspergillus niger* glucoamylase (*An*SBD) and *Rhizopus oryzae* glucoamylase (*Ro*SBD), respectively. The structure-function relationship of the SBD of *Rhizopus oryzae* glucoamylase (Ro*SBD*) using molecular modeling (Chou, W.I., Pai, T.W., Liu, S.H., Hsiung, B.K. & Chang, M.D. The family 21 carbohydrate-binding module of glucoamylase from Rhizopus oryzae consists of two sites playing distinct roles in ligand binding. Biochem J 396, 469-477 (2006)) and nuclear magnetic resonance (NMR) spectroscopy have identified (Liu, YN., Lai, Y.T., Chou, W.I., Chang, M.D. & Lyu, P.C. Solution structure of family 21 carbohydrate-binding module from Rhizopus oryzae glucoamylase. Biochem J 403, 21-30 (2007)). In addition, the maltoheptaose (G7) and β-cyclodextrin (βCD) binding sites on *Ro*SBD have been determined by X-ray crystallography (Tung, J.Y et al. Crystal structures of the starch-binding domain from Rhizopus oryzae glucoamylase reveal a polysaccharide-binding path. Biochem J 416, 27-36 (2008)).

The present invention further provides a pharmaceutical composition comprising a therapeutically or prophylactically effective amount of an antibody mimetic of carbohydrate binding module and a pharmaceutically acceptable carrier.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenecity, or other problems or complications, commensurate with a reasonable benefit/risk ratio.

The pharmaceutical compositions disclosed herein may be disclosed in unit-dosage forms or multiple-dosage forms. Unit-dosage forms, as used herein, refer to physically discrete units suitable for administration to human and animal subjects and packaged individually as is known in the art. Each unit-dose contains a predetermined quantity of the active ingredient(s) sufficient to produce the desired therapeutic effect, in association with the required pharmaceutical carriers or excipients. Examples of unit-dosage forms include ampoules, syringes, and individually packaged tablets and capsules. Unit-dosage forms may be administered in fractions or multiples thereof. A multiple-dosage form is a plurality of identical unit-dosage forms packaged in a single container to be administered in segregated unit-dosage form. Examples of multiple-dosage forms include vials, bottles of tablets or capsules, or bottles of pints or gallons.

The composition disclosed herein may be administered alone, or in combination with one or more other compounds disclosed herein, one or more other active ingredients. The pharmaceutical compositions that comprise a compound disclosed herein may be formulated in various dosage forms for oral, parenteral, and topical administration. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art.

The pharmaceutical compositions disclosed herein may be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

The pharmaceutical compositions disclosed herein may be disclosed in solid, semisolid, or liquid dosage forms for oral administration. As used herein, oral administration also include buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

The present invention further provides a method of detecting HIV of a sample, comprising adding an antibody mimetic of carbohydrate binding module (CBM) which specifically binds to an epitope on HIV glycoprotein to the sample; and detecing binding reaction between the antibody and the sample. In the method of detecting HIV, the CBM specifically binds to a glycan structure presents in the epitope of the glycoprotein.

The examples below are non-limiting and are merely representative of various aspects and features of the present invention.

### Example

### Example 1

### Structural correlation between SBDs and 2G12

### Structure-based multiple sequence alignment

Secondary structure prediction was performed using the Jpred server (Cuff, J.A., Clamp, M.E., Siddiqui, A.S., Finlay, M. & Barton, G.J. JPred: a consensus secondary structure prediction server. Bioinformatics 14, 892-893 (1998)) and the Network Protein Sequence Analysis (NPSA) server. The Jpred and NPSA servers provide consensus results from six (NNSSP, DSC, PREDATOR, MULPRED, ZPRED AND PHD) and twelve (SOPM, SOPMA, HNN, MLRC, DPM, DSC, GORI, GORII, GORIV, PHD, PREDATOR and SIMPA96) different methods respectively.

### Result

Examination of structure-function relationships between *Ro*SBD or *An*SBD and other protein domains was carried out by *in silico* screening using the DALI database (Holm, L., Kaariainen, S., Rosenstrom, P. & Schenkel, A. Searching protein structure databases with DaliLite v.3. Bioinformatics 24, 2780-2781 (2008)). In total, 555 and 535 structurally correlated proteins were retrieved for *Ro*SBD and *An*SBD, respectively, and were classified according to their functions. As expected, in both cases the most similar proteins (i.e., in top 200) were predominantly starch processing enzymes or CBMs, followed by the antibody family. In particular, *Ro*SBD and *An*SBD were correlated respectively with 158 and 57 immunoglobulin structures. Among these, *Ro*SBD resembled three sugar binding mAbs: 2G12, 2H1 and F22-4, which recognize the pathogenic microorganisms HIV, *Cryptococcus neoformans,* and *Shigella flexneri,* respectively. In addition, *Ro*SBD resembled 13 HIV neutralizing mAbs including 2F5 and FAB1583. Similarly, *An*SBD resembled two sugar binding antibodies: 2H1 and the single-chain antibody variable domain (scFv) of SE155-4 mAb; and two HIV binding mAbs, 2F5 and G3-519. The structural correlation among 2G12, *Ro*SBD, and *An*SBD allows ready deduction that these SBDs may have the potential to recognize the glycoprotein of HIV.

**Figure 1** shows the results of the structural comparison for *Ro*SBD, *An*SBD, and the V_{H} and V_{L} domains of 2G12 human mAb. Although the sequence identity between *Ro*SBD and *An*SBD, V_{H}, and V_{L} was as low as 8.3%, 9.5%, and 10.9%, respectively, the superimposed images indicate that the overall three dimensional structures are remarkably similar, among the pairs of structures, with an average root mean square deviation (RMSD) value of 1.4 Å **(****Figure 1a****),** 2.9 Å **(****Figure 1b****),** and 3.0 Å **(****Figure 1c****),** respectively. Although the multiple sequence alignment of *Ro*SBD, *An*SBD, V_{H}, and V_{L} in **Figure 1d** revealed low homology, the crucial ligand binding sites in the two SBDs and V_{H} were well conserved (Tung, J.Y et al. Crystal structures of the starch-binding domain from Rhizopus oryzae glucoamylase reveal a polysaccharide-binding path. Biochem J 416, 27-36 (2008)). The structural similarity among *Ro*SBD, *An*SBD, and the V_{H} and V_{L} domains of 2G12 may indicate a similar binding specificity for SBDs and 2G12.

### Example 2

### Functional correlation between SBDs and 2G12

### Microorganisms and plasmids

*Escherichia coli* Top10F' (Invitrogen) was used for plasmid manipulation, while the E. *coli* BL21-Gold (DE3) (Invitrogen) was used for protein expression. The vectors pET-23a(+) and pET-15b (Novagen), both containing a T7 promoter, were respectively used for recombinant *Ro*SBD and *An*SBD expression in E. *coli* cells. The forward primer **5'-CATATG**GCAAGTATTCCTAGCAGT-3' (SEQ ID NO. 1) and the reverse primer **5'-CTCGAG**TCATGTAGATACTTGGT 3' (SEQ ID NO. 2) for pET23a(+)-*Ro*SBD containing respectively *Nde*I and *Xho*I restriction sites, as well as the forward primer **5'-CATATG**AGCAAGACCAGCACCAGT-3' (SEQ ID NO. 3) and the reverse primer **5'-CTCGAG**CTACCGCCAGGTGT-3' (SEQ ID NO. 4) for pET15b-*An*SBD containing respectively *Nde*I and *Xho*I restriction sites were used for cloning and sequence analyses.

### Transformation and expression of SBDs

The pET expression vector was transformed into *E. coli* expression cells using heat shock method (Chou, W.I., Pai, T.W., Liu, S.H., Hsiung, B.K. & Chang, M.D. The family 21 carbohydrate-binding module of glucoamylase from Rhizopus oryzae consists of two sites playing distinct roles in ligand binding. Biochem J 396, 469-477 (2006)). One hundred microliters of competent cells and 100 ng DNA or 20 µL ligation mixture were mixed and then incubated on ice for 30 min. The cell was heated in the water bath at 42 °C for 30 to 60 sec, and subsequently placed on ice for more than 2 min. The transformants were spread onto Luria-Bertani (LB) agar plates containing 100 µg/mL ampicillin and incubated overnight at 37 °C for determination of number of transformants.

Protein expression was induced by addition of 0.5 mM isopropyl *β*-D-thiogalactoside (IPTG) (Promega) to the cultures and incubation at 20 °C with shaking at 250 x *g* for 16 h. The cells were harvested by centrifugation at 3,700 x g for 15 min at 4 °C.

### Purification of SBDs

Cell pellets of *E*. *coli* containing the recombinant protein were resuspened in 100 mL binding buffer (50 mM NaOAc, pH 5.5), lysed by homogenization (EmulsiFlex-C5 homogenizer, AVESTIN) and the cell debris was removed by centrifugation at 16,000 *x g* for 30 min at 4 °C. The resulting cell lysate was loaded onto the 5 mL amylose resin column (New England Biolabs). The column was then washed with 50 mL of binding buffer (50 mM NaOAc, pH 5.5) and the adsorbed protein was eluted with elution buffer (10 mM glycine-NaOH, pH 11). The purified SBD was concentrated by centrifugation in an ultrafiltration unit (Centricon-10, Millipore) and the buffer exchanged to 50 mM NaOAc, pH 5.5 (Lin, S.C. et al. CBM21 starch-binding domain: a new purification tag for recombinant protein engineering. Protein Expr Purif 65, 261-266 (2009)).

### Mass spectrometric analysis

Molecular mass determination of the recombinant proteins was performed by Liquid Chromatography/Mass Spectrometer (LC/MS) (Waters). The intact SBD (100 pmol) was acidified with 0.1% formic acid in 50% (v/v) acetonitrile and the data were acquired over the 800-1800 m/z range under normal scan resolution. The original electrospray mass spectrum with multiply charged ion series was deconvoluted to give a mass spectrum.

### Sandwich ELISA

Purified SBD (100 µL of a 100 nM solution) was diluted into SBD coating buffer (50 mM Tris-HCl buffer, pH 8) first and then pipeted into a 96-well ELISA plate (Greiner-Bio One), followed by incubation for 16 h at 4 °C. Similarly, 100 µL of 100 nM 2G12 mAb (Polymum Scientific) was diluted into 2G12 coating buffer (50 mM glycine-HCl buffer, pH 3) first and then pipeted into a second ELISA plate for 16 h at 4 °C. One each of the following four reagents (100 µL) was separately added to the SBD-coated or 2G12-coated ELISA plates, and allowed to react at 37 °C for 1 h: (i) HIV-PC (HIV antigen and antibody positive serum, ID# 9144532; SeraCare Life Sciences), (ii) 1.0 µg/mL Anti-*Ro*SBD mAb (produced by the authors and available upon request), (iii) HCV-PC (Anti-HCV Mixed Titer Performance Panel; member 10, ID# PHV205; SeraCare Life Sciences) and (iv) HTLV-PC (Anti-HTLV I/II Mixed Titer Performance Panel; member 10, ID# PRP206; SeraCare Life Sciences). The HIV-PC was confirmed as HIV antigen positive and anti-HIV antibody positive using an HIV p24 ELISA kit (Perkin Elmer) and an Anti-HIV 1+2 ELISA kit (Abbott Diagnostics) respectively, according to the manufacturers' instructions. Following this, the plates were washed with PBST (0.01 M Phosphate buffered saline plus 0.05% tween-20, pH 7.1) three times.

One each of the following four conjugate reagents were separately diluted into 0.05 µg/mL and added at 100 µL/well at 37 °C for 30 min: (i) HRP-conjugated recombinant gp120 antigen (General Biologicals), (ii) HRP-conjugated Anti-Mouse IgG (Jackson ImmunoResearch), (iii) HRP-conjugated HCV recombinant antigen (General Biologicals) and (iv) HRP-conjugated HTLV recombinant antigen (General Biologicals) followed by washing three times with PBST. The 3,3',5,5'-tetramethyl benzidine (General Biologicals) substrate was added at 100 µL/well and color development was allowed for 30 min at 37 °C. Color reaction was stopped by adding 100 µL/well of 2N sulfuric acid and the absorbance was measured at 450 nm using an Emax precision microplate reader (Molecular Devices). The cut-off value (COV) was defined as the OD of the negative control, (normal human serum) + 0.1. A sample exhibiting a cut-off index (COI) great than 1 was considered a positive, where COI = (OD of test sample)/COV

### Statistical analysis

Statistical analyses of data were performed by two-way ANOVA followed by unpaired two-tailed *t* test GraphPad Prism Version 4; GraphPad Software). P < 0.05 was considered significant.

### Result

A direct sandwich enzyme-linked immunosorbent assay (ELISA) was constructed employing an HIV-specific mAb or SBD-coated microtiter plates and horseradish peroxidase (HRP)-conjugated recombinant gp120; samples exhibiting a cut-off index (COI) > 1 were deemed positive. **Figure 1e** shows that the HIV antigen present in the HIV antigen and antibody positive control (HIV-PC) was clearly detected by anti-p24 mAb (N29), anti-gp41 mAb (AG10H9), anti-gp120 mAb (ED8.D4), and 2G12 as expected. Signals were observed in plates coated with either SBDs, which strongly suggests that *Ro*SBD and *An*SBD possess similar HIV detection capabilities.

**Figure 2a** revealed that *Ro*SBD binding occurred only when HIV-PC was tested. Negligible cross reaction with an anti-hepatitis C virus (HCV) mixed titer performance positive control (HCV-PC) or an anti-human T-lymphotropic virus (HTLV) mixed titer performance positive control (HTLV-PC) was observed. Moreover, HRP-conjugated HCV recombinant antigen, HRP-conjugated HTLV recombinant antigen, and HRP-conjugated anti-mouse IgG did not give significant signal **(****Figure 2****)** indicating that *Ro*SBD possesses specific recognition for HIV-PC, possibly due to binding to the HIV gp120 antigen.

**Figure 3a** shows that the maximal COI value for *An*SBD binding to HIV-PC was similar to that of *Ro*SBD, and no cross reactivity for *An*SBD binding to HCV-PC or HTLV-PC was observed. **Figures 3b** and **3c** suggest that AnSBD exhibited binding specificity to HIV-PC in the presence of HRP-conjugated recombinant gp120. In addition, there was no binding between mouse anti-RoSBD mAb and *An*SBD **(****Figure 3d****).**

### Example 3

### Specificity between RoSBD and AnSBD and HIV-1 glycoprotein

### Competitive ELISA of HIV mAbs and glycans

The following peptides and mAbs were used for the competitive ELISA experiment: (i) human IgG₁ secondary antibody ([2C11]; subclass-specific for human IgG₁ and no allotype restriction; Fc-region specific), (ii) HIV-1 p24 peptide ([N29], synthetic peptide corresponding to *N*-terminal residues 1-104 of HIV-1 p24), (iii) HIV-1 gp120 peptide ([ED8.D4]; synthetic peptide corresponding to residues 427-448 of Human HIV-1 BH8 isolate gp160), (iv) HIV-1 gp41 peptide ([AG10H9], synthetic peptide corresponding to residues 721-744 of HIV-1 gp160), (v) 2G12 mAb (recombinant human mAb against HIV-1 gp120), (vi) gp140 (HIV clade A, strain 92/UG/037). All were obtained from Abcam, except for 2G12 and gp140 which were purchased from Polymun Scientific.

For the ELISA experiment, 50 µL HIV-PC was mixed with an equal volume of 500 nM of each of (i) to (vi) above. The mixture was transferred to the *Ro*SBD or *An*SBD coated plate and incubated for 1 h at 37 °C followed by washing three times with PBST. The reaction was detected by adding 100 µL of 0.05 µg/mL HIV1 recombinant gp120 HRP-conjugate antigen. For the experiments to test glycan effects, 50 µL of 500 µM of each of the four samples, soybean agglutinin (SBA), maltoheptaose (G7), β-cyclodextrin (βCD) and mannose (Sigma-Aldrich), was mixed with an equal volume of HIV-PC. The mixture was transferred to the *Ro*SBD or *An*SBD coated plate and incubated for 1 h at 37 °C followed by washing three times with PBST. The reaction was detected by adding 100 µL of 0.05 µg/mL HRP-conjugated HIV1 recombinant gp120 antigen.

### Result

The specificity of molecular interaction between the two SBDs and HIV-1 was evaluated in the presence of competitors including mAbs, glycoproteins, and glycans. In **Figures 4a** and 4b, the presence of human IgG₁ (2C11) secondary antibody showed no inhibitory effect, indicating that no non-specific competition takes place. However, the COI values for HIV-PC binding to *Ro*SBD and *An*SBD significantly decreased to 72% and 60%, respectively, in the presence of recombinant HIV-1 gp140, a Chinese hamster ovary cell (CHO)-expressed envelope glycoprotein containing the same high mannose type glycan as gp120. This suggests molecular interactions between SBDs and HIV glycoprotein. In addition, the inhibition rate of the mAbs 2G12, anti-gp120 mAb, anti-gp41 mAb, and anti-p24 mAb competitors was 55%, 42%, 22%, and 13% respectively for *Ro*SBD-HIV PC binding, whereas that of *An*SBD was 33%, 18%, 6%, and 10% respectively. The evident reduction in SBD and HIV-PC binding when in competition with mAbs (for gp120) lends further support to the conclusion that the SBDs recognize HIV. The data strongly suggest specific interaction between each SBD and HIV gp120, presumably involving the glycan moiety on gp120.

Additionally, 500 µM tetrameric glycoprotein soybean agglutinin (SBA) possessing M9 moiety, maltoheptaose (G7), and β-cyclodextrin (βCD) were found to inhibit binding by 38%, 20% and 23%, as well as 16%, 13% and 11% for *Ro*SBD-HIV and *An*SBD-HIV respectively, although a mannose monosaccharide showed no inhibitory effect **(****Figure 5****).** These data imply that SBA and polysaccharide ligands of SBDs weakly compete for HIV binding.

### Example 4

### Comparative HIV-1 detection of SBDs and 2G12

To evaluate any practical application of SBDs for use in an HIV ELISA, a set of the HIV-1 incidence/prevalence performance panel samples (PRB601; SeraCare Life Sciences), containing 7 consensus incident and 8 consensus prevalent samples characterized to be HIV-1 positive, was used to compare the HIV detection performance among anti-p24, anti-gp41, anti-gp120, and 2G12 mAbs, as well as *Ro*SBD and *An*SBD.

### Result

**Figure 6** revealed that all coating materials were reactive and the two SBDs showed evident detection signals. Although similar detection profiles for the 15 samples were observed with either SBD or HIV-specific mAb-coated assay, 2G12 and *Ro*SBD showed relatively higher detection sensitivity; apparently, sample numbers 5, 6, 8, 10, 11, and 13 contained more HIV-1 antigens than the others. These data strongly supported the HIV-1 antigen recognition ability of our SBDs.

Furthermore, not only the detection patterns but also relative scale of the signals were quite similar among 2G12 **(****Figure 7a****),** *Ro*SBD **(****Figure 7b****),** and *An*SBD **(****Figure 7c****).** The detection rate of 2G12-, *Ro*SBD-, and *An*SBD-coated ELISA was as high as 93%, 93%, and 87%, respectively. It should be noted that samples No.1 and No.12 were not detected by *Ro*SBD and 2G12, respectively, although their HIV-1 RNA content was found to be 3 x 10⁴ and 2 x 10³ copies/mL respectively. The discrepancy between HIV antigen ELISA and HIV-1-RNA tests may arise from different detection targets or different expression level of surface glycoproteins.

### Example 5

### Specific mannan binding activity of RoSBD

### Glycan array screening of RoSBD

Glycan microarray analyses were conducted by the Consortium for Functional Glycomics (CFG), Core H facility (Raman, R. et al. Advancing glycomics: implementation strategies at the consortium for functional glycomics. Glycobiology 16, 82R-90R (2006)). The Mammalian Printed Array contained a total of 377 different natural and synthetic glycans. Briefly, Seventy microliters of *Ro*SBD (200 µg/mL) was applied to the printed surface of the array, coverslipped, and incubated in a dark humidified chamber at 37 °C for 1 h. After the incubation, the coverslip was removed and rinsed four times in TSM buffer (50 mM Tris-HCl, pH 7.5, 10 mM MgCl₂, and 0.5 M sucrose). Seventy microliters of anti-*Ro*SBD mAb was applied to the printed surface of the microarray and incubated in a humidified chamber at 37 °C for 1 h. To detect binding, the microarray was incubated with a secondary antibody, Alexa Fluor 488-conjugated goat anti-mouse IgG (Invitrogen) at a concentration of 5 µg/mL in PBS buffer at 37 °C for 1 h in a humidified chamber, followed by wash steps. The extent of binding was determined using a Perkin-Elmer Microarray XL4000 scanner and analyzed using Imagene (V.6) image analysis software. Complete glycan array data sets may be found in the CFG data archive under cfg_rRequest_1340.

### Magnetic reduction binding assay

For Magnetic Reduction (MR) measurement (MagQu), a solution containing homogeneously dispersed magnetic nanoparticles (MagQu) of 63.2 nm coated with hydrophilic surfactants (eg. dextran) was used. Under external multiple ac magnetic fields, the magnetic nanoparticles oscillate via magnetic interaction and show mixed-frequency magnetic susceptibility (χac), a magnetic property sensitive to the presence of molecules interacting with the particle surface. The χac value thus decreases in response to the association between magnetic nanoparticles and binding molecules in the test samples to give MR signal.

A ferrite solution containing a stoichiometric ratio of 1:2 ferrous sulfate hepta-hydrate (FeSO₄·7H₂O) and ferric chloride hexa-hydrate (FeCl₃·6H₂O) was mixed with an equal volume of aqueous dextran (Sigma-Aldrich) and dispersed in water at room temperature. The mixture was heated to 70-90 °C and titrated with 10 N NaOH (Showa chemical) to form Fe₃O₄ particles. Aggregates and excess unbound dextran were removed by centrifugation at 3,700 x g for 15 min at 25 °C and gel filtration chromatography (Sigma-Aldrich).

The magnetic fluid containing Fe₃O₄ magnetic nanoparticles was diluted to 0.3 emu/g (or 8.3 mg Fe/mL) with phosphate buffered saline (PBS, pH 7.4) prior to conjugation with purified *Ro*SBD (200 µL of 1.0 mg/mL) to the oxidized aldehyde groups on dextran to generate CH = N- linkages. Sixty microliters of 0.7 pM IgG1 (2C11), HIV-1-gp140, Man4 (as 6-aminohexyl glycoside (Man4-OR)), M9-Asn (Oda, Y. et al. Crocus sativus lectin recognizes Man3GlcNAc in the N-glycan core structure. J Biol Chem 275, 26772-26779 (2000)), or SBA with PBS (pH 7.4) solution was separated added into the glass tube and placed at 25 °C. Then another 60 µL magnetic reagent containing *Ro*SBD-coated nanoparticles was added into the glass tube and mixed for 15 sec at 25 °C. Finally, the mixture was detected using XacPro-S101 reader (MagQu) for measuring MR signals at 25 °C. As the association enabled magnetic nanoparticles became either larger or clustered, the reduced χac of the reagent was recorded.

### Result

**Figure 8a** clearly demonstrated two major signals detected by *Ro*SBD: glycan 54 FuC_{α(1,2)}Gal_{β(1,3)}GalNAc_{β(1,3)}Gal_{α}, and glycan 187 Man_{α(1,2)}Man_{α(1,2)}Man_{α(1,3)}Ma_{α}(Man4). (Man4). The former is associated with human blood type O antigen glycosylation (Korchagina, E.Y. et al. Design of the blood group AB glycotope. Glycoconj J 22, 127-133 (2005)), and the latter is a synthetic polysaccharide component of M9 in HIV gp120 (Wang, J., Li, H., Zou, G. & Wang, L.X. Novel template-assembled oligosaccharide clusters as epitope mimics for HIV-neutralizing antibody 2G12. Design, synthesis, and antibody binding study. Org Biomol Chem 5, 1529-1540 (2007)) as well as SBA. Such data provided an additional and independent line of evidence for interaction between *Ro*SBD and the HIV gp120, since Man4 recognizing the CD4 receptor on the host acts as the key component for HIV-1 entry. Interestingly, 2G12 has also been shown to bind to this Man4 structure of HIV-1 gp120, using glycan array analysis and other methods (Ji, X., Gewurz, H. & Spear, G.T. Mannose binding lectin (MBL) and HIV. Mol Immunol 42, 145-152 (2005) and Liu, W.T. et al. Identification and characterization of a novel fibril forming peptide in fungal starch binding domain. Biochem Biophys Res Commun 377, 966-970 (2008)).

The real-time glycan binding signal of *Ro*SBD was examined using a magnetic reduction (MR) assay (Hong C. Y, W.C.C., Chiu Y. C, Yang S.Y, Horng H.E, and Yang H.C. Magnetic susceptibility reduction method for magnetically labeled immunoassay. Applied Physics Letters 88, 21252 (2006)). A typical real-time magnetic response, χ_{ac}, of the mixture containing *Ro*SBD-coated magnetic beads and phosphate-buffered saline (PBS) or human IgG₁ is shown in **Figure 8b** (solid dots and solid triangles), indicating that a non-specific interaction between *Ro*SBD and human IgG₁ secondary antibody was not present, as also demonstrated in **Figure 4****.** In the presence of a trace amount of HIV-gp140 (0.7 pM) however, a clearly reduced MR signal (open squares) was detected (Δχ_{ac} of 3.5%). Interestingly, the presence of the same molar concentration of Man4 (as 6-aminohexyl glycoside, Man4-OR), M9-Asn (from SBA) and SBA **(****Figure 9****)** resulted in similar MR signals (crosses and open triangles; Δχ_{ac} of 3.6%, 3.6% and 4.0 respectively).

Characteristic signal reduction in the MR experiment were clearly observed for HIV-gp140, Man4-OR, M9-Asn, and the whole SBA tests, providing strong evidence for a direct molecular interaction between *Ro*SBD and high mannose-type glycans. As expected from its tetravalent feature, SBA reacted much faster than the others.

Taken together, these results demonstrate that *Ro*SBD and *An*SBD bind to the native HIV antigen in HIV-PC through a novel protein-glycan interaction, specifically targeting the Man4 moiety on HIV-1 gp120.

### Example 6

### Structure resemblance between SBDs and Man4-binding proteins and Mapping Man4 binding sites on RoSBD

### Molecular modeling of the putative Man4 binding site

Modeling of the putative Man4 binding site of *Ro*SBD was undertaken by superimposing the three-dimensional structures of the *Ro*SBD complex (PDB ID: 2V8M) and the 2G12-Man4 complex (PDB ID: 1ZLS) using the Dali server. The monosaccharide Man4 was in order to find putative binding residues on *Ro*SBD within a 5 Å radius. In addition, using the Man4 coordinates from the 2G12-Man4 complex, Man4 was fitted into the G7 binding site of the *Ro*SBD-G7 structure, such that the most conserved hexose ring interacted with binding sites I and II. Residues within a 5Å radius of Man4 in the predicted *Ro*SBD-Man4 complex were labeled.

### Site-directed mutagenesis of RoSBD

*Ro*SBD and its mutants were generated using the PCR-based QuikChange® site-directed mutagenesis method (Stratagene) with pET23a-*Ro*SBD as the template. Primer pairs used to amplify the SBD derivatives were designed and listed in **Table 1.** The PCR mixture containing 10 ng template, 0.625 µL of each primer (10 µM), 2.5 µL reaction buffer (10x), 3.125 µL dNTPs (2.5 mM), 0.25 µL *Pfu* Turbo DNA polymerase (20 U/µL) (Stratagene) in a final volume of 25 µL with ddH₂O. Thermal cycle condition used in this study was 1 cycle of 95 °C for 5 min and 20 cycles of 95 °C for 1 min (denaturation), 45 °C to 65 °C for 30 sec (annealing) and 68 °C for 8 min (extension) and finally 1 cycle of 68 °C for 10 min. PCR reaction was performed in a thermal cycler (9700, Applied Biosystems). The sequence of each mutant plasmid was verified by DNA sequencing analysis.

**Table 1. Oligonucleotide primers for site-directed mutagenesis. Mutations that generated an alanine codon are underlined. Primers denoted - F' and - R' comprised the sense and antisense strands, respectively.**

| | |
|---|---|
| N29A-F' | 5'-ATTTATGTCAAGGCCATTGCTTACTCCAAG-3' |
| | (SEQ ID NO. 5) |
| N29A-R' | 5'-GGAGTAAGCAATGGCCTTGACATAAATTTTTCC-3' |
| | (SEQ ID NO. 6) |
| Y32A-F' | 5'-GTCAAGAACATTGCTGCCTCCAAGAAAGTTACT-3' |
| | (SEQ ID NO. 7) |
| Y32A-R' | 5'-AGTAACTTTCTTGGAGGCAGCAATGTTCTTGAC-3' |
| | (SEQ ID NO. 8) |
| K34A-F' | 5'-CATTGCTTACTCCGCGAAAGTTACTGTAGTC-3' |
| | (SEQ ID NO. 9) |
| K34A-R' | 5'-GACTACAGTAACTTTCGCGGAGTAAGCAAT-3' |
| | (SEQ ID NO. 10) |
| W47A-F' | 5'-GATGGCTCTGACAACGCGAATAATAATGGAAAC-3' |
| | (SEQ ID NO. 11) |
| W47A-R' | 5'-GTTTCCATTATTATTCGCGTTGTCAGAGCCATC-3' |
| | (SEQ ID NO. 12) |
| F58A-F' | 5'-CATCATTGCTGCTTCTGCCTCTG-3' |
| | (SEQ ID NO. 13) |
| F58A-R' | 5'-AGAGATAGGGCCAGAGGCAGAAG-3' |
| | (SEQ ID NO. 14) |
| Y67A-F' | 5'-CTCTGGATCAAATGCCGAATACTGG-3' |
| | (SEQ ID NO. 15) |
| Y67A-R' | 5'-ATGTCCAGTATTCGGCATTTGATCC-3' |
| | (SEQ ID NO. 16) |
| Y83A-F' | 5'-GGTACCATTAAGTATGAAGTC-3' |
| | (SEQ ID NO. 17) |
| Y83A-R' | 5'-GACTTCATACTTAATGGCGAACTCCTTGATACC-3' |
| | (SEQ ID NO. 18) |
| Y93A-F' | 5'-GTCAGTGGAAAAACAGCCTATGATAACAACAAT-3' |
| | (SEQ ID NO. 19) |
| Y93A-R' | 5'-ATTGTTGTTATCATAGGCTGTTTTTCCACTGAC-3' |
| | (SEQ ID NO. 20) |
| Y94A-F' | 5'-GTGGAAAAACATACGCTGATAACAACAATTC-3' |
| | (SEQ ID NO. 21) |
| Y94A-R' | 5'-GAATTGTTGTTATCAGCGTATGTTTTTCCAC-3' |
| | (SEQ ID NO. 22) |

### Result

Currently, available at the CFG Core H database, are 10 Man4-binding proteins, of which two are calcium-dependent lectins possessing mainly beta-rich structures, Cv lectin (CV-IIL) derived from *Chromobacterium violaceum* and BclA lectin from *Burkholderia cenocepacia,* belonging to the same homologous CATH superfamily (2.60.120.400) as *An*SBD. These lectins are virulence factors discovered in pathogenic bacteria with the highest affinity for methyl α-D-fucoside (Emau, P. et al. Griffithsin, a potent HIV entry inhibitor, is an excellent candidate for anti-HIV microbicide. J Med Primatol 36, 244-253 (2007)) and methyl α-D-mannoside. Their possible roles might be related to host recognition, adhesion, and biofilm formation (Fromme, R. et al. A monovalent mutant of cyanovirin-N provides insight into the role of multiple interactions with gp120 for antiviral activity. Biochemistry 46, 9199-9207 (2007)). Their high structural similarity provides strong evidence for functional correlation between the two fungal SBDs and Mannan-binding proteins.

**Figure 10a** showed the electrostatic potential of the complexes 2G12V_{H}-Man4 and *Ro*SBD-G7. The hydroxyl groups of Man4 form hydrogen bonds to Thr33 and Asp100, the glycan ring III of Man4 is bound to Tyr56 of 2G12V_{H} domain (left panel), while G7 circles around Tyr32 of *Ro*SBD, and may be stabilized by neighboring Trp47, Tyr83, and Tyr 94 (right panel). Interestingly, the orientation of glycan rings II and III of G7 in four different conformations has been shown to be well conserved²⁹, and the hydrophobic phenyl ring of Phe58 in *Ro*SBD provides a favorable stacking interaction with the glycan ring III of the glucosyl unit of G7.

To predict the putative Man4 binding site on *Ro*SBD, the Man4 structure was isolated from its complex with V_{H} domain and superimposed it on *Ro*SBD at a corresponding loop, as shown in **Figure 1b****,** using the Man4 coordinates in the PDB ID: 1ZLS structure (Calarese, D.A. et al. Dissection of the carbohydrate specificity of the broadly neutralizing anti-HIV-1 antibody 2G12. Proc Natl Acad Sci U S A 102, 13372-13377 (2005)) as the central point to search for neighboring residues within a radius of less than 5 Å on *Ro*SBD. Using this approach, residues Tyr32 and Ser33 were predicted to be involved in Man4 binding **(****Figure 10b****,** left panel). It has been shown that there were two carbohydrate-binding sites, sites I and II, in the *Ro*SBD-G7 complex, site I being at Trp47 around loop β34, and site II being at Tyr32 around loop β23 (Tung, J.Y. et al. Crystal structures of the starch-binding domain from Rhizopus oryzae glucoamylase reveal a polysaccharide-binding path. Biochem J 416, 27-36 (2008)). Replacement of G7 with Man4 revealed that residues Asn29, Tyr32, Lys34, Trp47, Phe58, Glu68, Tyr83, and Tyr94 may be involved in binding of Man4 (Figure **10b****,** right panel).

**Figure 10c** illustrated the effect of alanine substitution in positions Gln29, Tyr32, Ser33, Lys34, Trp47, Phe58, Tyr83, Tyr93 and Tyr94. Interestingly, N29A, Y32A, W47A, and F58A significantly decreased HIV-binding activity to 29%, 43%, 40%, and 53%, respectively, and K34A, Y83A, and Y94A showed moderate reductions in HIV-binding. The S33A and Y93A mutants, however, retained the same HIV binding activity as wild type *Ro*SBD, as opposed to the binding model in Figure **10a****.** The aromatic residues Y32, W47, and F58 have been well defined as crucial ligand binding sites of *Ro*SBD, Y83 and Y94 hold the ligand from the neighboring *Ro*SBD, and K34 could stabilize D68²⁷; this strongly supported the prediction that Man4 could be recognized by the G7 ligand binding sites, as illustrated in Figure **10b****.** Taken together, the *in silico* assessment of structure-function relationship between the starch-binding CBMs and HIV gp120 glycan-binding 2G12 has been confirmed by *in vitro* analyses.

### Example 7

### Inhibition of HIV-1 infection by RoSBD and their cellular toxicities

### Cells, virus strain and antiviral assay

The following cells and virus strain were obtained from the AIDS Research and Reference Reagent Program, Division of AIDS, National Institute of Allergy and Infectious Disease: H9 cells and virus strain of HIV-1_{RTMF} (AZT-resistant virus). H9 cells were grown and maintained in RPMI 1640 medium supplemented with 10% fetal bovine serum, 100 units/ml penicillin and 100 mg/mL streptomycin. H9 cells were incubated with HIV-1 virus at 37 °C for two hours. H9 cells were suspended in culture medium at 1x10⁵ cells per ml and infected with HIV at a multiplicity of infection of 0.1. After infection, the cells were washed twice with PBS followed by culture medium. The cell suspension (100 µL) was added to each well of a 96-well plate and then increasing concentrations of SBD (0, 1.25, 2.5, 5, 10, 20, 40 & 80 µM) were added to the infected cells. After 4-day incubation, the cells were sub-cultured with fresh culture medium containing appropriate concentrations of SBD and incubated an additional 4 days. The activity of SBD against viral replication of HIV-1_{RTMF} virus in H9 cells was tested. Antiviral activity was determined by using the HIV-1 p24 antigen ELISA. (di Marzo Veronese, F. et al. Monoclonal antibodies specific for p24, the major core protein of human T-cell leukemia virus type III. Proc Natl Acad Sci U S A 82, 5199-5202 (1985)).

### Cytotoxicity Assay

The cytotoxicity of SBD against H9 cells was analyzed using MTT assay. Briefly, exponentially growing H9 cells were seeded onto 96-well plates at a density of 3×10⁴ cells/well and incubated for 24 h at 37 °C prior to drug exposure. On the day of treatment, a series of SBD concentrations (0, 1.25, 2.5, 5, 10 and 20 µM) were used to test their cytotoxicity in H9 cells as prescribed (Abd-Elazem, I.S., Chen, H.S., Bates, R.B. & Huang, R.C. Isolation of two highly potent and non-toxic inhibitors of human immunodeficiency virus type 1 (HIV-1) integrase from Salvia miltiorrhiza. Antiviral Res 55, 91-106 (2002)). After 8 days of incubation, 50 µl of MTT was added (1 mg/mL final concentration) and the plates were incubated at 37 °C for 4 h to allow MTT to form formazan crystals by reacting with metabolically active cells. The formazan crystals were solubilized with DMSO. The absorbance of each well was measured in a microtiter reader at optical density 540 nm. The reaction was specific; no significant amounts of formazan could be detected with dead cells.

### Result

Viral replication in the absence and presence of different *Ro*SBD concentrations was measured by using the HIV-1 p24 antigen ELISA after eight days. The antiviral activity of *Ro*SBD against HIV-1 infection in H9 cells was determined as mentioned in the materials and methods. *Ro*SBD inhibited AZT-resistant virus with IC₅₀ value of 4.5 µM as calculated from the dose response curve **(****Figure 11****).** A series of *Ro*SBD concentrations ranging from 1.25 to 80 µM was used for testing the cytotoxicity of *Ro*SBD in H9 cells. Using MTT assay to determine the viability of H9 cell in culture, it proved that the cells remained viable during the entire culture period except at the higher concentrations of SBD (40 and 80 µM), where H9 cells were dead. For 20 µM concentration, the viability of cells was about 79 % while at 10 µM was 88 %.

## Claims

1. An antibody mimetic, which is starch binding domain (SBD) that specifically binds to an epitope on HIV glycoprotein, for use in inhibiting prophylactically or therapeutically HIV infection of a subject.

2. The antibody mimetic for use in accordance with claim 1, wherein the SBD specifically binds to a glycan structure present in the epitope of the HIV glycoprotein.

3. The antibody mimetic for use in accordance with claim 1, wherein the HIV glycoprotein is HIV1-gp120.

4. The antibody mimetic for use in accordance with claim 2, wherein the glycan structure is *N*-linked high mannose glycan Man₉GlcNAc₂.

5. The antibody mimetic for use in accordance with claim 1, wherein the SBD is a member selected from the group consisting of CBM families 20, 21, 25, 26, 34, 41, 45, 48 and 53.

6. The antibody mimetic for use in accordance with claim 5, wherein the SBD is CBM family 20.

7. The antibody mimetic for use in accordance with claim 5, wherein the SBD is CBM family 21.

8. The antibody mimetic for use in accordance with claim 1, wherein the SBD is derived from *Rhizopus oryzae* Glucoamylase (*Ro*SBD) or *Aspergillus niger* glucoamylase (*An*SBD).

9. The antibody mimetic for use in accordance with claim 1, wherein the subject is human.

10. A pharmaceutical composition for use in inhibiting prophylactically or therapeutically HIV infection of a subject, comprising a therapeutically or prophylactically effective amount of an antibody mimetic (immunoglobulin-like) and a pharmaceutically acceptable carrier, wherein the antibody mimetic is starch binding domain (SBD).

11. The composition for use in accordance with claim 10, wherein the SBD specifically binds to a mannose-rich glycan structure present in the epitope of the HIV glycoprotein.

12. The composition for use in accordance with claim 10, wherein the SBD is derived from *Rhizopus oryzae* glucoamylase (*Ro*SBD) or *Aspergillus niger* glucoamylase (*An*SBD).

13. The composition for use in accordance with claim 10, which is in a form of a tablet, a capsule or a suspension.

14. A method of detecting HIV of a sample, comprising adding an antibody mimetic which specifically binds to an epitope on HIV glycoprotein to the sample; and detecing binding reaction between the antibody and the sample, wherein the antibody mimetic is starch binding domain (SBD).

15. The method of claim 14, wherein the SBD specifically binds to a mannose-rich glycan structure present in the epitope of the glycoprotein.

16. The method of claim 14, wherein the glycoprotein is HIV1-gp120.

17. The method of claim 15, wherein the glycan structure is *N*-linked high mannose glycan Man₉GlcNAc₂.

18. The method of claim 14, wherein the SBD is a member selected from the group consisting of CBM families 20, 21, 25, 26, 34, 41, 45, 48 and 53.

19. The method of claim 18, wherein the SBD is CBM family 20.

20. The method of claim 18, wherein the SBD is CBM family 21.

21. The method of claim 14, wherein the SBD is derived from *Rhizopus oryzae* glucoamylase (*Ro*SBD) or *Aspergillus niger* glucoamylase (*An*SBD).

22. The method of claim 14, wherein the sample is isolated from human.

## Patentansprüche

1. Antikörpermimetikum, welches eine Stärkebindungsdomain (SBD) darstellt, die spezifisch an ein Epitop auf dem HIV-Glykoprotein bindet, zur Verwendung in der prophylaktischen oder therapeutischen Inhibierung einer HIV-Infektion eines Patienten.

2. Antikörpermimetikum zur Verwendung nach Anspruch 1, worin die SBD spezifisch an eine Glykanstruktur bindet, die in dem Epitop des HIV-Glykoproteins vorliegt.

3. Antikörpermimetikum zur Verwendung nach Anspruch 1, worin das HIV-Glykoprotein HIV1-gp120 ist.

4. Antikörpermimetikum zur Verwendung nach Anspruch 2, worin die Glykanstruktur *N-*verbundenes Hochmannoseglykan Man₉GlcNAc₂ ist.

5. Antikörpermimetikum zur Verwendung nach Anspruch 1, worin die SBD ein Mitglied darstellt, ausgewählt aus der Gruppe, bestehend aus CBM-Familien 20, 21, 25, 26, 34, 41, 45, 48 und 53.

6. Antikörpermimetikum zur Verwendung nach Anspruch 5, worin die SBD die CBM-Familie 20 ist.

7. Antikörpermimetikum zur Verwendung nach Anspruch 5, worin die SBD die CBM-Familie 21 ist.

8. Antikörpermimetikum zur Verwendung nach Anspruch 1, worin die SBD abgeleitet ist von *Rhizopus oryzae* Glucoamylase (*Ro*SBD) oder *Aspergillus niger* Glucoamylase (*An*SBD).

9. Antikörpermimetikum zur Verwendung nach Anspruch 1, worin der Patient ein Mensch ist.

10. Pharmazeutische Zusammensetzung zur Verwendung in der prophylaktischen oder therapeutischen Inhibierung einer HIV-Infektion eines Patienten, umfassend eine therapeutisch oder prophylaktisch wirksame Menge eines Antikörpermimetikums (Immunoglobulin-ähnlich) und einen pharmazeutisch annehmbaren Träger, worin das Antikörpermimetikum eine Stärkebindungsdomain (SBD) darstellt.

11. Zusammensetzung zur Verwendung nach Anspruch 10, worin die SBD spezifisch an eine mannosereiche Glykanstruktur bindet, die in dem Epitop des HIV-Glykoproteins vorliegt.

12. Zusammensetzung zur Verwendung nach Anspruch 10, worin die SBD abgeleitet ist von *Rhizopus oryzae* Glucoamylase (*Ro*SBD) oder *Aspergillus niger* Glucoamylase (*An*SBD).

13. Zusammensetzung zur Verwendung nach Anspruch 10, welche in Form einer Tablette, einer Kapsel oder einer Suspension vorliegt.

14. Verfahren zur Detektierung von HIV in einer Probe, umfassend die Zugabe eines Antikörpermimetikums, welches spezifisch an ein Epitop auf dem HIV-Glykoprotein bindet, zu der Probe; und Detektieren einer Bindungsreaktion zwischen dem Antikörper und der Probe, worin das Antikörpermimetikum eine Stärkebindungsdomain (SBD) darstellt.

15. Verfahren nach Anspruch 14, worin die SBD spezifisch an eine mannosereiche Glukanstruktur bindet, die in dem Epitop des Glykoproteins vorliegt.

16. Verfahren nach Anspruch 14, worin das Glykoprotein HIV1-gp120 ist.

17. Verfahren nach Anspruch 15, worin die Glykanstruktur *N*-verbundenes Hochmannoseglykan Man₉GlcNAc₂ ist.

18. Verfahren nach Anspruch 14, worin die SBD ein Mitglied ist, ausgewählt aus der Gruppe, bestehend aus den CBM-Familien 20, 21, 25, 26, 34, 41, 45, 48 und 53.

19. Verfahren nach Anspruch 18, worin die SBD die CBM-Familie 20 ist.

20. Verfahren nach Anspruch 18, worin die SBD die CBM-Familie 21 ist.

21. Verfahren nach Anspruch 14, worin die SBD abgeleitet ist von *Rhizopus oryzae* Glucoamylase (*Ro*SBD) oder *Aspergillus niger* Glucoamylase (*An*SBD).

22. Verfahren nach Anspruch 21, worin die Probe vom Menschen isoliert wird.

## Revendications

1. Un mimétique d'anticorps, qui est le domaine de liaison de l'amidon (SBD) qui se lie spécifiquement à un épitope sur la glycoprotéine du VIH, pour usage dans l'inhibition prophylactique ou thérapeutique de l'infection par le VIH d'un sujet.

2. Le mimétique d'anticorps pour usage selon la revendication 1, le SBD se liant spécifiquement à une structure de glycane présente dans l'épitope de la glycoprotéine du VIH.

3. Le mimétique d'anticorps pour usage selon la revendication 1, la glycoprotéine du VIH étant la gp120 de VIH-1.

4. Le mimétique d'anticorps pour usage selon la revendication 2, la structure de glycane étant le glycane riche en mannose Man₉GlcAc₂ lié par N.

5. Le mimétique d'anticorps pour usage selon la revendication 1, le SBD étant un élément choisi parmi le groupe constitué des familles CBM 20, 21, 25, 26, 34, 41, 45, 48 et 53.

6. Le mimétique d'anticorps pour usage selon la revendication 5, le SBD étant la famille de CBM 20.

7. Le mimétique d'anticorps pour usage selon la revendication 5, le SBD étant la famille de CBM 21.

8. Le mimétique d'anticorps pour usage selon la revendication 1, le SBD étant dérivé de la glucoamylase de *Rhizopus oryzae* (*Ro*SBD) où de la glucoamylase d'*Aspergillus niger* (*An*SBD).

9. Le mimétique d'anticorps pour usage selon la revendication 1, le sujet étant humain.

10. Une composition pharmaceutique pour usage dans l'inhibition prophylactique ou thérapeutique de l'infection du VIH d'un sujet, comprenant une quantité thérapeutiquement ou prophylactiquement effective d'un mimétique d'anticorps (de la nature de l'immunoglobuline) et un excipient pharmaceutiquement acceptable, le mimétique d'anticorps étant le domaine de liaison de l'amidon (SBD).

11. La composition pour usage selon la revendication 10, le SBD se liant spécifiquement à une structure de glycane riche en mannose présente dans l'épitope de la glycoprotéine du VIH.

12. La composition pour usage selon la revendication 10, le SBD étant dérivé de la glucoamylase de *Rhizopus oryzae* (*Ro*SBD) où de la glucoamylase d'*Aspergillus niger* (*An*SBD).

13. La composition pour usage selon la revendication 10, qui est sous une forme d'un cachet, d'une capsule ou d'une suspension.

14. Un procédé de détection du VIH d'un échantillon, comprenant l'ajout d'un mimétique d'anticorps qui se lie spécifiquement à un épitope sur la glycoprotéine du VIH à l'échantillon ; et la détection de la réaction de liaison entre l'anticorps et l'échantillon, le mimétique d'anticorps étant le domaine de liaison de l'amidon (SBD).

15. Le procédé de la revendication 14, le SBD se liant spécifiquement à une structure de glycane riche en mannose et qui est présente dans l'épitope de la glycoprotéine.

16. Le procédé de la revendication 14, la glycoprotéine étant la gp120 de VIH-1.

17. Le procédé de la revendication 15, la structure de glycane étant le glycane riche en mannose Man₉GlcNAc₂ lié par N.

18. Le procédé de la revendication 14, le SBD étant un élément choisi parmi le groupe constitué des familles CBM 20, 21, 25, 26, 34, 41, 45, 48 et 53.

19. Le procédé de la revendication 18, le SBD étant la famille de CBM 20.

20. Le procédé de la revendication 18, le SBD étant la famille de CBM 21.

21. Le procédé de la revendication 14, le SBD étant dérivé de la glucoamylase de *Rhizopus oryzae* (*Ro*SBD) où de la glucoamylase d'*Aspergillus niger* (*An*SBD).

22. Le procédé selon la revendication 14, l'échantillon étant isolé de l'humain.
